# EUROPEAN PATENT SPECIFICATION

(11) **EP 4 487 789 B1**
(45) Date of publication and mention of the grant of the patent: **07.10.2026**
(21) Application number: 23779336.9
(22) Date of filing: 08.03.2023
(51) Int. Cl.: A61B 10/00, A61B 5/107, G16H 50/20, A61B 5/00, G16H 30/40, G16H 40/63, G16H 40/67

(54) **COMPUTER PROGRAM, INFORMATION PROCESSING METHOD, AND INFORMATION PROCESSING DEVICE**
COMPUTERPROGRAMM, INFORMATIONSVERARBEITUNGSVERFAHREN UND INFORMATIONSVERARBEITUNGSVORRICHTUNG
PROGRAMME INFORMATIQUE, PROCÉDÉ ET DISPOSITIF DE TRAITEMENT D'INFORMATIONS

(30) Priority: 28.03.2022 JP 2022052275
(43) Date of publication of application: 08.01.2025
(73) Proprietor: Terumo Kabushiki Kaisha, Tokyo 151-0072 (JP)
(72) Inventor: HONMA, Yasuyuki, Ashigarakami-gun, Kanagawa 259-0151 (JP); NISHIMURA, Toshihiko, Ashigarakami-gun, Kanagawa 259-0151 (JP)
(74) Representative: Prüfer & Partner mbB Patentanwälte · Rechtsanwälte
(86) International application number: PCT/JP2023/008702
(87) International publication number: WO 2023/189309

(56) References cited:
- WO-A1-2018/102579
- WO-A1-2022/030592
- WO-A1-2022/054687
- CN-A- 112 768 065
- JP-A- 2011 209 916
- JP-A- 2011 209 916
- JP-A- 2013 097 588
- JP-A- 2020 107 038
- KR-A- 20190 135 598
- US-A1- 2010 172 567

## Description

### Technical Field

The present disclosure relates to a computer program, and an information processing device that estimate presence or absence of neuropathy of a subject.

### Background Art

Patent Literature 1 proposes a stroke determination device that acquires a face image including a face of a subject, determines presence or absence of facial nerve palsy of the subject on the basis of the face image using a learned model subjected to deep learning in advance, presents an inquiry item related to stroke to the subject, acquires an answer to the inquiry item, and determines a possibility of stroke of the subject on the basis of the presence or absence of facial nerve palsy and the answer.

### Citation List

### Patent Literature

Patent Literature 1: JP 2020-199072 A
Patent literature 2 : WO 2018/102579

### Summary of Invention

### Technical Problem

In order to determine the presence or absence of facial nerve palsy on the basis of the face image of the subject imaged by a camera as in the stroke determination device disclosed in Patent Literature 1, it is difficult to accurately determine the presence or absence of facial nerve palsy without using a face image obtained by imaging the face of the subject from the front.

The present disclosure is achieved in view of such circumstances, and an object thereof is to provide a computer program, and an information processing device that can be expected to accurately estimate presence or absence of neuropathy of a subject on the basis of face information acquired in a direction other than a front view.

### Solution to Problem

A computer program according to one embodiment allows a computer to execute processing of acquiring face information of a subject detected by a sensor, generating face structure information of the subject on the basis of the acquired face information, complementing missing face information of the subject with respect to the acquired face information on the basis of the generated face structure information, and estimating presence or absence of neuropathy of the subject on the basis of the complemented face information.

### Advantageous Effects of Invention

In a case of one embodiment, it can be expected to accurately estimate the presence or absence of neuropathy of the subject on the basis of the face information acquired in a direction other than the front view.

### Brief Description of Drawings

Fig. 1 is a schematic diagram for illustrating an overview of an information processing system according to this embodiment.
Fig. 2 is a block diagram illustrating a configuration of a server device according to a first embodiment.
Fig. 3 is a block diagram illustrating a configuration of a terminal device according to this embodiment.
Fig. 4 is a flowchart for illustrating a procedure of pre-processing performed by the information processing system according to the first embodiment.
Fig. 5 is a schematic diagram for illustrating the pre-processing performed by the information processing system according to the first embodiment.
Fig. 6 is a flowchart illustrating a procedure of processing of estimating presence or absence of neuropathy performed by the server device according to this embodiment.
Fig. 7 is a flowchart illustrating a procedure of face image complementing processing performed by the server device according to the first embodiment.
Fig. 8 is a schematic diagram for illustrating a method of estimating the presence or absence of neuropathy performed by the server device according to this embodiment.
Fig. 9 is a schematic diagram illustrating an example of a notification screen of an estimation result of neuropathy.
Fig. 10 is a schematic diagram for illustrating a configuration of a learning model included in a server device according to a second embodiment.
Fig. 11 is a schematic diagram illustrating an example of shape information output by a shape estimation model.
Fig. 12 is a flowchart illustrating a procedure of face image complementing processing performed by the server device according to the second embodiment.

### Description of Embodiments

A specific example of an information processing system according to an embodiment of the present disclosure will be hereinafter described with reference to the drawings. Note that, the present disclosure is not limited by these examples but is recited in claims, and is intended to include all changes within the meaning and scope equivalent to claims.

### <System Configuration>

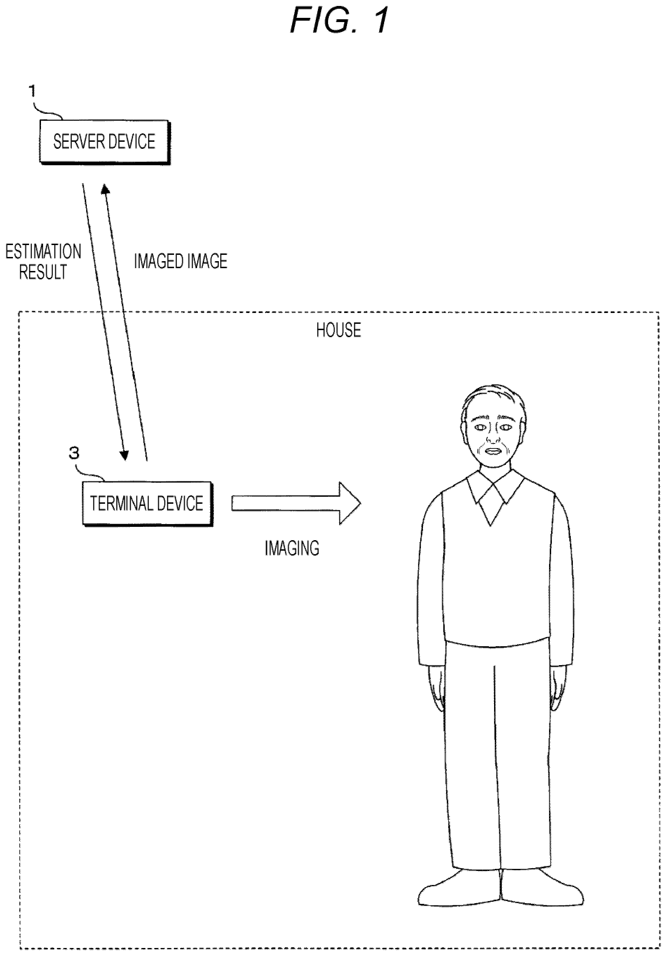

Fig. 1 is a schematic diagram for illustrating an overview of an information processing system according to this embodiment. The information processing system according to this embodiment includes a server device 1 that performs processing of estimating presence or absence of neuropathy of a subject, and one or a plurality of terminal devices 3 that images a face image of the subject required for estimation processing performed by the server device 1. In the information processing system according to this embodiment, the terminal device 3 is installed in a house where the subject lives (for example, a living room, a dining room or the like), a nursing care facility, a medical facility or the like. The terminal device 3 is equipped with a camera, periodically images the subject in the house, and transmits the imaged image to the server device 1.

The server device 1 performs processing of estimating the presence or absence of neuropathy such as stroke (cerebrovascular disorder), cerebral infarction, or facial nerve palsy for the subject on the basis of the imaged image of the subject acquired from the terminal device 3. The server device 1 according to this embodiment estimates the presence or absence of neuropathy on the basis of the face of the subject included in the imaged image. For example, in a case where it is estimated that the subject has neuropathy, the server device 1 transmits this estimation result to the terminal device 3. The terminal device 3 that receives the estimation result can notify the subject or another user (a family member, a medical worker or the like) related to the subject of a risk of neuropathy by performing processing such as displaying a warning message on a display unit or outputting voice, for example. Note that, also in a case where it is estimated that the subject does not have neuropathy, the server device 1 may transmit the fact as the estimation result to the terminal device 3.

The information processing system according to this embodiment may estimate the presence or absence of neuropathy on the basis of the image of the subject imaged by the camera by the terminal device 3, and, in a case where it is estimated that there is neuropathy, detect information of the subject by a sensor different from the camera mounted on the terminal device 3, for example, a ranging sensor, a microphone (microphone) or the like, and further estimate the presence or absence of neuropathy on the basis of the detected information. For example, the terminal device 3 that receives, from the server device 1, the estimation result that there is neuropathy on the basis of the image imaged by the camera detects information by a sensor different from the camera, and transmits the detected information to the server device 1. The server device 1 further performs processing of estimating the presence or absence of neuropathy of the subject on the basis of the information received from the terminal device 3. Examples of information detection of the subject include, for example, the detection of information such as biological information abnormality, facial palsy, mental abnormality, fall, shaking or shivering, weakness, or speech abnormality. Specific symptoms of biological information abnormality include abnormalities in pulse, heart rate variability, respiration, blood oxygen concentration, or blood pressure variability.

For example, in a case where it is determined that there is neuropathy on the basis of the image imaged by the camera, and it is determined that there is neuropathy on the basis of the information detected by another sensor, the server device 1 finally transmits the estimation result that there is neuropathy for the subject to the terminal device 3. For example, in a case where it is determined that there is neuropathy on the basis of the image imaged by the camera, but it is determined that there is no neuropathy on the basis of the information detected by another sensor, the server device 1 finally transmits the estimation result that there is no neuropathy for the subject to the terminal device 3.

Note that, additional estimation regarding the presence or absence of neuropathy may be performed on the basis of not the information detected by the sensor different from the camera but the image of the subject imaged by the camera. In this case, the additional estimation is performed by a method different from the determination of the presence or absence of neuropathy based on the face of the subject, for example, a method of determining the presence or absence of neuropathy on the basis of an entire body of the subject. The additional estimation is not necessarily performed, and the server device 1 may only estimate the presence or absence of neuropathy on the basis of the face of the subject imaged by the camera of the terminal device 3.

In a case where it is estimated that the subject has neuropathy, the information processing system according to this embodiment may perform a diagnostic test on the subject in order to grasp, for example, a degree of symptoms of neuropathy. For example, the server device 1 transmits information for performing the diagnostic test for neuropathy based on a determination index such as Cincinnati Prehospital Stroke Scale (CPSS), National Institute of Health Stroke Scale (NIHSS), or Kurashiki Prehospital Scale (KPSS) to the terminal device 3 of the subject determined to have neuropathy. The terminal device 3 performs the diagnostic test on the basis of the information received from the server device 1, and transmits information obtained from the subject to the server device 1. The server device 1 determines regarding one or a plurality of test items included in the diagnostic test on the basis of the information received from the terminal device 3, and transmits a result of the diagnostic test to the terminal device 3. The terminal device 3 displays the result of the diagnostic test received from the server device 1, and notifies the subject of the same. Note that, the diagnostic test is not necessarily performed.

In the information processing system according to this embodiment, the server device 1 performs various types of processing in order to accurately perform first estimation processing regarding the presence or absence of neuropathy, that is, the estimation of the presence or absence of neuropathy based on the face image of the subject imaged by the camera of the terminal device 3. The server device 1 examines bilateral symmetry of the face of the subject on the basis of the face image of the subject imaged by the camera of the terminal device 3, thereby estimating the presence or absence of neuropathy. The server device 1 compares the face image in normal time of the subject registered in advance with the face image of the subject imaged by the camera of the terminal device 3, and estimates the presence or absence of neuropathy on the basis of a difference therebetween.

Note that, the estimation of the presence or absence of neuropathy described above is based on the premise that the face of the subject imaged by the camera of the terminal device 3 is of a front view. The information processing system according to this embodiment images the subject by the camera of the terminal device 3 installed in the house of the subject, and cannot always image the face of the subject from the front. Therefore, in the information processing system according to this embodiment, in a case where the face image of the subject imaged by the camera of the terminal device 3 is imaged in a direction other than the direction from the front (for example, from obliquely right front, obliquely left front or the like), and an entire face of the subject is not imaged in the imaged image, the server device 1 complements a portion not imaged (missing portion) to generate the image in the front view of the face of the subject, and estimates the presence or absence of neuropathy by the above-described method.

### <Device Configuration>

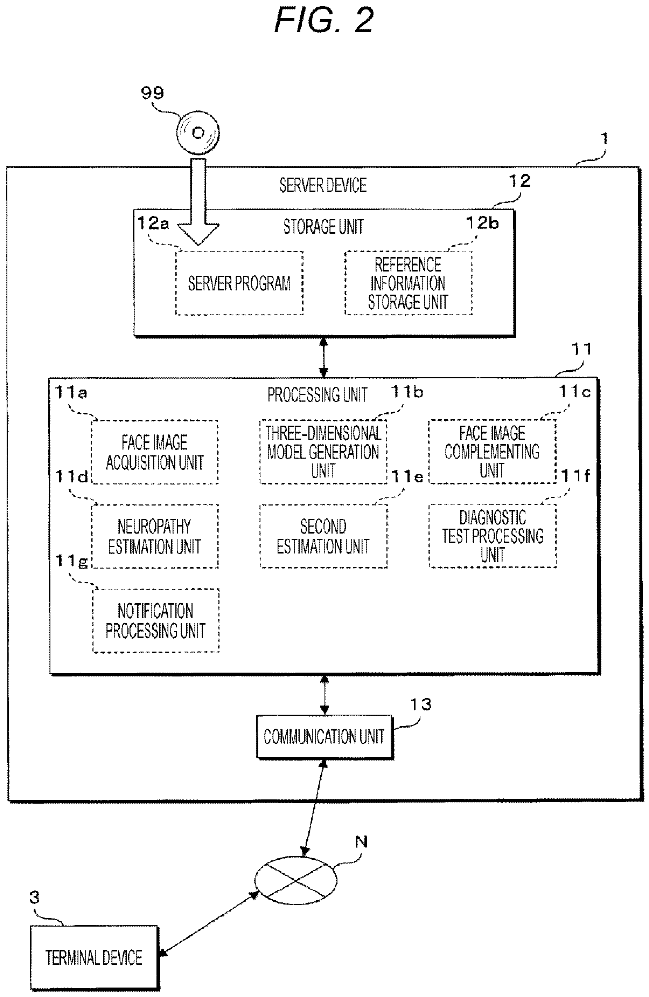

Fig. 2 is a block diagram illustrating a configuration of the server device 1 according to a first embodiment. The server device 1 according to this embodiment includes a processing unit 11, a storage unit (storage) 12, a communication unit (transceiver) 13 and the like. Note that, in this embodiment, the description will be given assuming that the processing is performed by one server device, but a plurality of server devices may perform the processing in a distributed manner.

The processing unit 11 includes an arithmetic processing unit such as a central processing unit (CPU), a micro-processing unit (MPU), a graphics processing unit (GPU), or a quantum processor, a read only memory (ROM), a random access memory (RAM) and the like. By reading and executing a server program 12a stored in the storage unit 12, the processing unit 11 performs various pieces of processing such as processing of estimating the presence or absence of neuropathy of the subject on the basis of the imaged image acquired from the terminal device 3 and processing of performing the diagnostic test on the subject determined to have neuropathy.

The storage unit 12 is formed using, for example, a large-capacity storage device such as a hard disk. The storage unit 12 stores various programs executed by the processing unit 11 and various data required for processing of the processing unit 11. In this embodiment, the storage unit 12 is provided with a reference information storage unit 12b that stores the server program 12a executed by the processing unit 11 and information used for processing of estimating the presence or absence of neuropathy.

In this embodiment, the server program (computer program, program product) 12a is provided in a form recorded in a recording medium 99 such as a memory card or an optical disk, and the server device 1 reads the server program 12a from the recording medium 99 and stores the same in the storage unit 12. Note that, the server program 12a may be written in the storage unit 12 at a manufacturing stage of the server device 1, for example. For example, the server device 1 may acquire the server program 12a distributed by another remote server device and the like through communication. For example, the server program 12a recorded in the recording medium 99 may be read by a writing device, and written in the storage unit 12 of the server device 1. The server program 12a may be provided in a form of distribution via a network, or may be provided in a form recorded in the recording medium 99.

The reference information storage unit 12b stores, as reference information, information regarding the face of the subject (face in normal time) acquired and/or generated in advance. The information stored in the reference information storage unit 12b can include, for example, information of an imaged image obtained by imaging the face of the subject from the front, and a three-dimensional model of the face of the subject.

The communication unit 13 communicates with various devices via a network N including a mobile phone communication network, a wireless local area network (LAN), the Internet and the like. In this embodiment, the communication unit 13 communicates with one or a plurality of terminal devices 3 via the network N. The communication unit 13 transmits the data given from the processing unit 11 to another device and gives the data received from another device to the processing unit 11.

Note that, the storage unit 12 may be an external storage device connected to the server device 1. The server device 1 may be a multi-computer including a plurality of computers or may be a virtual machine virtually constructed by software. The server device 1 is not limited to the above-described configuration, and may include, for example, a reading unit that reads information stored in a portable storage medium, an input unit that receives an operation input, a display unit that displays an image or the like.

In the server device 1 according to this embodiment, the processing unit 11 reads and executes the server program 12a stored in the storage unit 12, so that a face image acquisition unit 11a, a three-dimensional model generation unit 11b, a face image complementing unit 11c, a neuropathy estimation unit 11d, a second estimation unit 11e, a diagnostic test processing unit 11f, a notification processing unit 11g and the like are implemented in the processing unit 11 as software functional units. Note that, in this drawing, functional units regarding processing of estimating the presence or absence of neuropathy of the subject are illustrated as the functional units of the processing unit 11, and functional units regarding other processing are not illustrated.

The face image acquisition unit 11a performs processing of acquiring the face image of the subject imaged by the terminal device 3 by communicating with the terminal device 3 by the communication unit 13. For example, in a case where the entire body of the subject is imaged in the image imaged by the terminal device 3, the face image acquisition unit 11a may perform processing of detecting the face of the subject from the imaged image and extracting a partial image of the detected face. Note that, the processing of extracting the face image of the subject from the imaged image may be performed not by the server device 1 but by the terminal device 3, and in this case, the server device 1 can acquire the face image of the subject extracted from the imaged image from the terminal device 3.

The three-dimensional model generation unit 11b performs processing of generating a (latest) three-dimensional model of the face of the subject on the basis of the face image of the subject acquired by the face image acquisition unit 11a and the three-dimensional model of the subject stored in the reference information storage unit 12b.

In the information processing system according to this embodiment, imaging, measurement and the like of the face of the subject are performed in advance, and the three-dimensional model of the face of the subject is created in advance and stored in the reference information storage unit 12b of the server device 1. For example, the face of the subject is imaged from a plurality of angles, and two-dimensional images of the face of the subject from various angles are collected. A surface shape of the face of the subject is measured by a ranging sensor using infrared rays, ultrasonic waves or the like, and a shape model in which a shape of the face of the subject is reproduced in a three-dimensional virtual space is generated. By pasting the collected two-dimensional image on the shape model, the three-dimensional model serving as a reference of the face of the subject is generated. The server device 1 stores in advance the generated three-dimensional model and the two-dimensional images obtained by imaging the face of the subject from a plurality of angles when creating the three-dimensional model in the reference information storage unit 12b. In the following description, the three-dimensional model of the face of the subject stored in the reference information storage unit 12b is referred to as a reference three-dimensional model, and the two-dimensional image is referred to as a reference face image. Note that, instead of generating the shape model on the basis of a measurement result of the ranging sensor, the shape model may be generated on the basis of the two-dimensional image obtained by imaging the face of the subject from one or a plurality of angles. For example, a learned learning model of a face mesh might be used to generate the three-dimensional shape model from the two-dimensional image. The learning model of the face mesh is a machine learning model that detects key points (feature points) of a face from an image, and can output hundreds of feature points from a human face in three-dimensional coordinates.

Note that, as the reference three-dimensional model of the face of the subject, a plurality of models is preferably generated for a plurality of expressions, or a model capable of changing the expression is preferably generated. Therefore, it is preferable that the face of the subject is imaged and the surface shape is measured in various expressions of the subject. In this embodiment, it is preferable that a bilaterally asymmetric expression is included in the various expressions.

The three-dimensional model generation unit 11b pastes the face image of the subject acquired by the face image acquisition unit 11a on the reference three-dimensional model of the face of the subject stored in the reference information storage unit 12b, thereby generating the three-dimensional model of the face of the subject. At that time, for example, the three-dimensional model generation unit 11b compares the face image to be pasted with the face image pasted on a surface of the reference three-dimensional model, selects the reference three-dimensional model having the closest expression, and pastes the face image on the reference three-dimensional model. Therefore, the generated three-dimensional model is the model on which the face image of the subject imaged by the terminal device 3 is pasted, and is the model of the face image pasted on the reference three-dimensional model as for a portion of the face missing in the image imaged by the terminal device 3.

The face image complementing unit 11c performs processing of complementing a missing portion of the image obtained by imaging the face of the subject on the basis of the three-dimensional model of the face of the subject generated by the three-dimensional model generation unit 11b. The face image complementing unit 11c converts the generated three-dimensional model of the face into the two-dimensional image in the front view, that is, generates a two-dimensional image imaged by a virtual camera arranged in front of the three-dimensional model of the face in the three-dimensional virtual space, thereby acquiring the face image in the front view of the subject in which the missing portion in the image imaged by the terminal device 3 is complemented.

The neuropathy estimation unit 11d performs processing of estimating the presence or absence of neuropathy of the subject on the basis of the face image of the subject complemented by the face image complementing unit 11c. Note that, in a case where the face image of the subject acquired from the terminal device 3 is of the front view, the neuropathy estimation unit 11d may estimate the presence or absence of neuropathy of the subject on the basis of the image acquired from the terminal device 3 without performing the above-described complementing processing.

In this embodiment, the neuropathy estimation unit 11d extracts various feature points such as positions of the eyes, mouth, forehead, cheek and the like, and angles of the mouth corner and eyebrow from the face image of the subject, for example, and compares the extracted feature points on the right and left sides of the face to examine symmetry. For example, the neuropathy estimation unit 11d can calculate a displacement amount between right and left sides regarding various features of the face and estimate that there is neuropathy in a case where the calculated displacement amount exceeds a predetermined threshold.

For example, the neuropathy estimation unit 11d may compare a latest face image of the subject with a past face image and estimate the presence or absence of neuropathy on the basis of a difference therebetween. For this purpose, the server device 1 stores, in the reference information storage unit 12b, a face image (information of features extracted from the face image or the three-dimensional model generated from the face image may be used) imaged in advance in a normal state (a state without neuropathy) of the subject. The neuropathy estimation unit 11d extracts various features from the face image acquired from the terminal device 3 (alternatively, a face image obtained by complementing the face image acquired from the terminal device 3), and compares the features with the features extracted from the face image in the normal state stored in the reference information storage unit 12b. The neuropathy estimation unit 11d can calculate a displacement amount regarding the features in both face images and estimate that there is neuropathy in a case where the calculated displacement amount exceeds a predetermined threshold.

In this embodiment, the neuropathy estimation unit 11d compares the latest face image of the subject with the past face image separately for a right half and a left half of the face, and estimates that the subject has neuropathy in a case where the displacement amount exceeds a threshold on either one of the right and left sides. In a case where the displacement amount exceeds the threshold on both the right and left sides of the face and in a case where the displacement amount does not exceed the threshold on both the right and left sides of the face, the neuropathy estimation unit 11d estimates that the subject has no neuropathy. Note that, in a case where the displacement amount exceeds the threshold on both the right and left sides of the face, the neuropathy estimation unit 11d may estimate that there is neuropathy.

In a case where the neuropathy estimation unit 11d estimates that the subject has neuropathy, the second estimation unit 11e additionally estimates the presence or absence of neuropathy of the subject using a sensor different from the camera included in the terminal device 3. In the information processing system according to this embodiment, the shape of the face of the subject is measured using the ranging sensor included in the terminal device 3, the server device 1 acquires the measurement result from the terminal device 3, and the second estimation unit 11e checks bilateral symmetry as for the shape of the face of the subject or compares the same with the shape in normal time, thereby estimating the presence or absence of neuropathy of the subject. Note that, the additional estimation by the second estimation unit 11e may be performed using any sensor included in the terminal device 3, and the estimation may be performed by any method on the basis of the information acquired by the sensor.

In a case where the neuropathy estimation unit 11d and the second estimation unit 11e estimate that the subject has neuropathy, the diagnostic test processing unit 11f performs processing for performing the diagnostic test of the subject using the terminal device 3. In this embodiment, the diagnostic test processing unit 11f performs a stroke diagnostic test based on a determination index such as CPSS, NIHSS, or KPSS. In this embodiment, the diagnostic test might be performed, for example, by displaying a message of a question on the display unit of the terminal device 3 and receiving an answer by character input, voice input or the like from the subject. For example, the diagnostic test might be performed by displaying a message and the like requesting to make a predetermined movement, expression or the like on the display unit of the terminal device 3 and acquiring an image of a state in which the subject makes requested movement, expression or the like imaged by the camera. Note that, the method of performing the diagnostic test described above is merely an example, and there is no limitation; the diagnostic test processing unit 11f may perform any diagnostic test.

The diagnostic test processing unit 11f transmits information of the question, request or the like regarding the diagnostic test to the terminal device 3. On the basis of this information, the terminal device 3 outputs the message of the question, request or the like regarding the diagnostic test, receives the answer from the subject, images the movement or the like, and transmits information of the received answer, the imaged image or the like to the server device 1. The diagnostic test processing unit 11f of the server device 1 that receives the information regarding the diagnostic test from the terminal device 3 determines the presence or absence, the degree and the like of neuropathy of the subject on the basis of the received information.

The notification processing unit 11g performs processing of notifying of the estimation result of the neuropathy estimation unit 11d, the estimation result of the second estimation unit 11e, and/or a diagnosis result of the diagnostic test processing unit 11f. The notification processing unit 11g notifies the subject by transmitting information of the estimation result, the diagnosis result or the like to the terminal device 3. The notification processing unit 11g may notify another user related to the subject such as a family member on the basis of information such as an e-mail address or a telephone number registered in advance, for example.

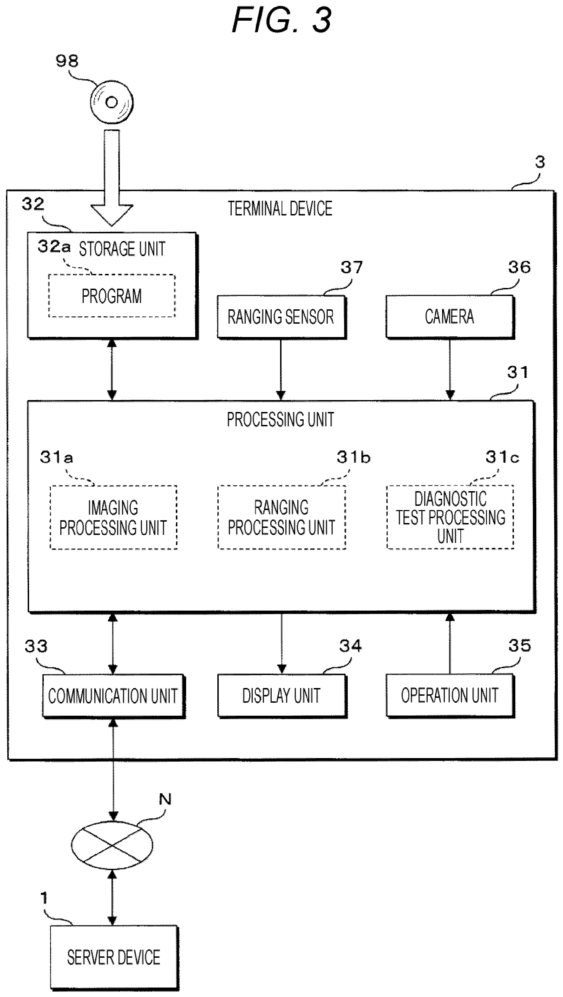

Fig. 3 is a block diagram illustrating a configuration of the terminal device 3 according to this embodiment. The terminal device 3 according to this embodiment includes a processing unit 31, a storage unit (storage) 32, a communication unit (transceiver) 33, a display unit (display) 34, an operation unit 35, a camera 36, a ranging sensor 37 and the like. The terminal device 3 is a device installed in the house and the like where the subject, the presence or absence of neuropathy of whom is estimated, lives. The terminal device 3 may be, for example, a device fixedly installed in the house and the like, or a portable device such as a smartphone or a tablet terminal device may be mounted on a stand and the like.

The processing unit 31 includes an arithmetic processing unit such as a CPU or an MPU, a ROM, a RAM and the like. The processing unit 31 reads and executes a program 32a stored in the storage unit 32, thereby performing various pieces of processing such as processing of imaging the subject by the camera 36, processing of detecting information regarding the subject by the ranging sensor 37, and processing of inputting and outputting information for the diagnostic test to and from the subject.

The storage unit 32 is formed by using, for example, a nonvolatile memory element such as a flash memory or a storage device such as a hard disk. The storage unit 32 stores various programs executed by the processing unit 31 and various data required for processing of the processing unit 31. In this embodiment, the storage unit 32 stores the program 32a executed by the processing unit 31. In this embodiment, the program 32a is distributed by a remote server device and the like, and the terminal device 3 acquires the same by communication and stores the same in the storage unit 32. Note that, the server program 32a may be written in the storage unit 32 at a manufacturing stage of the terminal device 3, for example. For example, the program 32a recorded in a recording medium 98 such as a memory card or an optical disk may be read by the terminal device 3 to be stored in the storage unit 32. For example, the program 32a recorded in the recording medium 98 may be read by a writing device, and written in the storage unit 32 of the terminal device 3. The program 32a may be provided in a form of distribution via a network, or may be provided in a form recorded in the recording medium 98.

The communication unit 33 communicates with various devices via the network N including the mobile phone communication network, the wireless LAN, the Internet and the like. In this embodiment, the communication unit 33 communicates with the server device 1 via the network N. The communication unit 33 transmits the data given from the processing unit 31 to another device and gives the data received from another device to the processing unit 31.

The display unit 34 is formed using a liquid crystal display and the like, and displays various images, characters and the like on the basis of processing of the processing unit 31. The operation unit 35 receives an operation of the user and notifies the processing unit 31 of the received operation. For example, the operation unit 35 receives the operation of the user by an input device such as a mechanical button or a touch panel and the like provided on a surface of the display unit 34. For example, the operation unit 35 may be an input device such as a mouse and a keyboard, and these input devices may be detachable from the terminal device 3.

The camera 36 includes an imaging element such as a charge coupled device (CCD) image sensor or a complementary metal oxide semiconductor (CMOS) image sensor. The camera 36 provides data of an image (moving image) imaged by the imaging element to the processing unit 31. Note that, the camera 36 may be built in the terminal device 3 or may be detachable from the terminal device 3.

The ranging sensor 37 is a sensor that emits, for example, infrared rays, ultrasonic waves, electromagnetic waves or the like, and detects reflected waves of them to measure a distance to the subject. As the ranging sensor 37, for example, a sensor referred to as light detection and ranging (LiDAR) might be used. In this embodiment, the ranging sensor 37 is used for additional estimation processing performed in a case where it is estimated that there is neuropathy by the processing of estimating the presence or absence of neuropathy on the basis of the face image of the subject imaged by the camera 36. The shape of the face of the subject can be measured by the ranging sensor 37, and the server device 1 can estimate the presence or absence of neuropathy of the subject on the basis of the measurement result. Note that, the sensor included in the terminal device 3 for the additional estimation processing is not limited to the ranging sensor 37, and may be, for example, a sensor (microphone) that detects voice, or any other sensor.

In the terminal device 3 according to this embodiment, the processing unit 31 reads and executes the program 32a stored in the storage unit 32, so that an imaging processing unit 31a, a ranging processing unit 31b, the diagnostic test processing unit 31c and the like are implemented in the processing unit 31 as software functional units.

The imaging processing unit 31a performs processing of imaging the subject by the camera 36 and transmitting the obtained imaged image to the server device 1. The imaging processing unit 31a repeatedly images at a cycle of several times to several tens of times per second, for example. The imaging processing unit 31a continuously images by the camera 36 regardless of whether the subject is in the house, and transmits an image obtained by the imaging to the server device 1. Note that, the imaging processing unit 31a may perform, for example, processing of detecting a person from the image obtained by imaging, processing of detecting a face of a person, processing of specifying who the person imaged in the image and the like, select an image in which the face of the subject is imaged, and transmit the image to the server device 1. The imaging processing unit 31a may extract a partial image in which the face of the subject is imaged from the image imaged by the camera 36 and transmit the extracted partial image to the server device 1. In a case where a human sensor and the like is mounted on the terminal device 3, for example, the imaging processing unit 31a may image only in a case where there is a person around the terminal device 3.

The ranging processing unit 31b performs processing of measuring the surface shape of the face of the subject by measuring the distance to the face of the subject by the ranging sensor 37. In this embodiment, the measurement by the ranging processing unit 31b is not always performed, but is started in a case where an instruction to perform the additional estimation processing is given from the server device 1. For example, the ranging processing unit 31b grasps a position of the face of the subject on the basis of the image imaged by the camera 36, extracts information corresponding to the position of the face of the subject from the information of the distance measured by the ranging sensor 37, and transmits the extracted information to the server device 1 as the information of the surface shape of the face of the subject.

The diagnostic test processing unit 31c outputs the message and the like to the subject and receives the input from the subject, thereby performing the diagnostic test on neuropathy. For example, the diagnostic test processing unit 31c displays a message of a question given from the server device 1 on the display unit 34, receives an input of an answer to the question from the subject by the operation unit 35, and transmits the received answer to the server device 1. Note that, the output of the question and the input of the answer may be performed by voice input/output. For example, the diagnostic test processing unit 31c displays, on the display unit 34, a message regarding a request for movement, expression or the like given from the server device 1, images the movement, expression or the like made by the subject in response to the request by the camera 36, and transmits an imaged image (moving image or still image) to the server device 1.

### <Pre-processing>

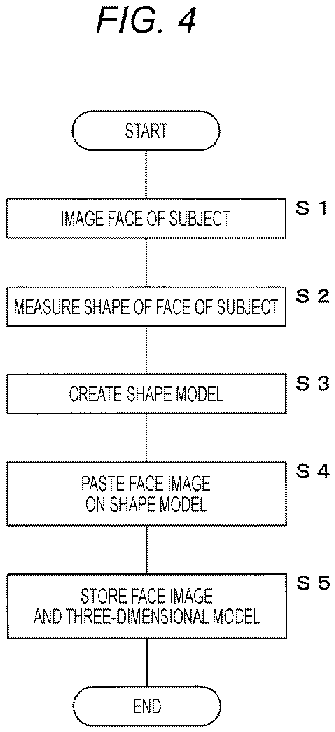

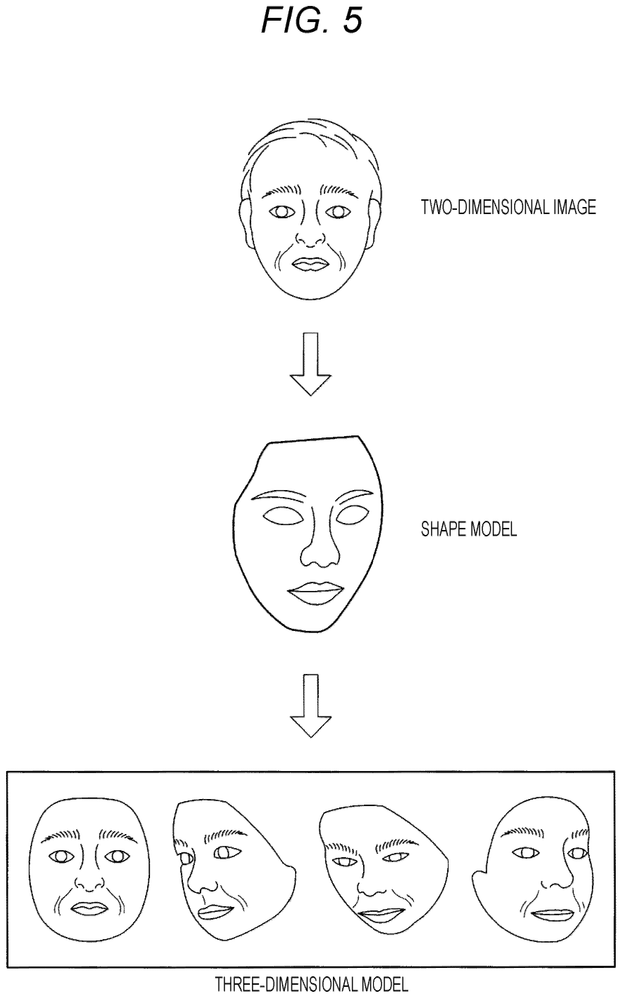

In the information processing system according to the first embodiment, face information in the normal state (a state without neuropathy) of the subject is acquired in advance as pre-processing. Fig. 4 is a flowchart for illustrating a procedure of the pre-processing performed by the information processing system according to the first embodiment. Fig. 5 is a schematic diagram for illustrating the pre-processing performed by the information processing system according to the first embodiment. Note that, the pre-processing may be performed by the server device 1, may be performed by the terminal device 3, or may be performed by using one or a plurality of other devices. In this embodiment, the pre-processing is performed using the server device 1 and the terminal device 3.

In the pre-processing, the server device 1 images the face of the subject, the presence or absence of neuropathy of whom is predicted, in the normal state using the camera 36 of the terminal device 3, for example (step S1). At that time, for example, by manually or automatically moving the terminal device 3 with respect to the face of the subject or by moving the face of the subject with respect to the camera 36 of the terminal device 3, a plurality of imaged images obtained by imaging the face of the subject in a plurality of directions is acquired. At that time, for example, a message is displayed on the display unit 34 of the terminal device 3 to request the subject to make various expressions, and a plurality of imaged images obtained by imaging the face of various expressions is acquired. It is preferable that a bilaterally asymmetric expression is included in the various expressions. An upper part of Fig. 5 illustrates an example of the face image of the subject obtained by the processing at step S1.

The server device 1 measures a three-dimensional shape of the face of the subject using, for example, the ranging sensor 37 of the terminal device 3 (step S2). Also for this measurement, it is preferable to perform measurement with the various expressions of the subject. The imaging at step S1 and the measurement at step S2 may be performed simultaneously. The server device 1 creates the three-dimensional shape model obtained by reproducing the face of the subject in the three-dimensional virtual space on the basis of a measurement result at step S2 (step S3). Note that, the shape model created by the server device 1 at step S3 is a model that reproduces the shape (structure) of the face of the subject, and a color, a pattern or the like of the surface of the face is not reproduced. A middle part of Fig. 5 illustrates an example of the shape model created by the processing at step S3.

Next, the server device 1 generates the three-dimensional model of the face of the subject by pasting the face image of the subject imaged at step S1 on a surface of the shape model created at step S3 (step S4). The three-dimensional model generated at step S4 is a model a direction of which can be changed in the three-dimensional virtual space. Moreover, a plurality of three-dimensional models is preferably generated in association with a plurality of expressions of the subject, or the three-dimensional model is preferably a model capable of changing the expression in the three-dimensional virtual space. A lower part of Fig. 5 illustrates an example of the three-dimensional model generated by the processing at step S4, and a plurality of images corresponding to a case where the three-dimensional model is changed in a plurality of directions is arranged in a horizontal direction.

The server device 1 stores the face image acquired at step S1 and the three-dimensional model generated at step S4 in the reference information storage unit 12b of the storage unit 12 (step S5), and ends the pre-processing. These pieces of information stored in the reference information storage unit 12b of the server device 1 are used for processing of complementing the face image of the subject imaged by the terminal device 3 when the processing of estimating the presence or absence of neuropathy of the subject is performed.

### <Estimation Processing>

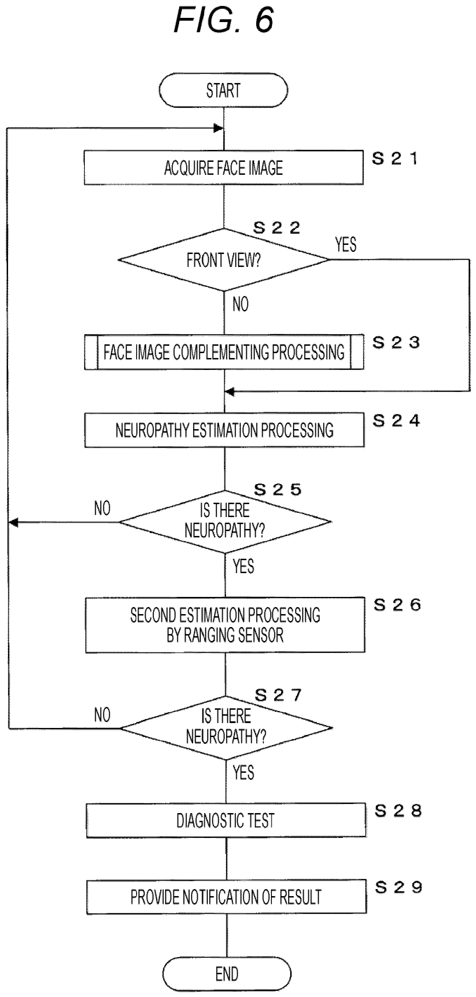

In the information processing system according to this embodiment, the terminal device 3 installed in the house of the subject continuously images by the camera 36, and continuously transmits the image of the subject (the face image of the subject) obtained by the imaging to the server device 1. The server device 1 acquires the face image of the subject imaged by the terminal device 3 and performs processing of estimating the presence or absence of neuropathy of the subject. Fig. 6 is a flowchart illustrating a procedure of the processing of estimating the presence or absence of neuropathy performed by the server device 1 according to this embodiment.

The face image acquisition unit 11a of the processing unit 11 of the server device 1 according to this embodiment communicates with the terminal device 3 by the communication unit 13, and acquires the face image of the subject imaged by the camera 36 by the terminal device 3 (step S21). The face image acquisition unit 11a determines whether the face image acquired at step S21 is the image in the front view obtained by imaging the face of the subject from the front (step S22). In a case of the image in the front view (S22: YES), the processing unit 11 advances the processing to step S24. In a case of not the image in the front view (S22: NO), the processing unit 11 performs face image complementing processing (step S23) and advances the processing to step S24. Note that, the face image complementing processing performed at step S23 will be described later.

The neuropathy estimation unit 11d of the processing unit 11 performs processing of estimating the presence or absence of neuropathy of the subject on the basis of the face image acquired from the terminal device 3 or the face image obtained by correcting the face image (step S24). At that time, the neuropathy estimation unit 11d compares the right half and the left half of the face of the subject imaged in the face image, and estimates the presence or absence of neuropathy on the basis of bilateral symmetry. The neuropathy estimation unit 11d compares the face image acquired from the terminal device 3 or the face image obtained by correcting the face image with the face image of the subject in the normal state stored in the reference information storage unit 12b, and estimates the presence or absence of neuropathy on the basis of the difference therebetween.

The processing unit 11 determines whether it is estimated that the subject has neuropathy by the neuropathy estimation processing at step S24 (step S25). In a case where it is estimated that there is no neuropathy (S25: NO), the processing unit 11 returns the processing to step S21 and repeatedly performs the above-described processing. In a case where it is estimated that there is neuropathy (S25: YES), the second estimation unit 11e of the processing unit 11 performs the second estimation processing by the ranging sensor 37 included in the terminal device 3 (step S26). In the second estimation processing, the second estimation unit 11e transmits, for example, a command to perform measurement by the ranging sensor 37 to the terminal device 3, and acquires a measurement result (face information) of the surface shape of the face of the subject by the ranging sensor 37 from the terminal device 3. Similarly to a case of the estimation processing on the basis of the face image, for example, the second estimation unit 11e can estimate the presence or absence of neuropathy of the subject by the ranging sensor 37 by a method such as the estimation of the presence or absence of neuropathy on the basis of bilateral symmetry as for the surface shape of the face of the subject, or the estimation of the presence or absence of neuropathy on the basis of the comparison with the surface shape of the face of the subject in the normal state.

The processing unit 11 determines whether it is estimated that the subject has neuropathy by the second estimation processing at step S26 (step S27). In a case where it is estimated that there is no neuropathy (S27: NO), the processing unit 11 returns the processing to step S21 and repeatedly performs the above-described processing. In a case where it is estimated that there is neuropathy (S27: YES), the diagnostic test processing unit 11f of the processing unit 11 performs the diagnostic test regarding neuropathy through question and answer with the subject via the terminal device 3 (step S28). The diagnostic test processing unit 11f transmits, for example, a message of a question for the subject to the terminal device 3, thereby allowing the terminal device 3 to output the message and acquiring an answer received by the terminal device 3 from the subject. The diagnostic test processing unit 11f transmits, for example, a message requesting the subject to make the movement or expression to the terminal device 3 and outputs the message, and acquires an image obtained by imaging the movement or expression made by the subject for the message by the camera 36 from the terminal device 3. The diagnostic test processing unit 11f collects information regarding the subject by performing these pieces of processing a plurality of times, and determines the presence or absence, the degree and the like of neuropathy on the basis of the index such as CPSS, NIHSS, or KPSS, for example.

The notification processing unit 11g of the processing unit 11 transmits information regarding a result of the estimation processing at step S24, a result of the estimation processing at step S26, and/or a result of the diagnostic test at step S28 to the terminal device 3, thereby notifying the subject of the result of the estimation and diagnosis regarding neuropathy (step S29), and ends the processing. Note that, the notification processing unit 11g may notify a terminal device other than the terminal device 3 installed in the home of the subject, for example, a terminal device used by a family member of the subject, a terminal device used by a doctor in charge of the subject or the like.

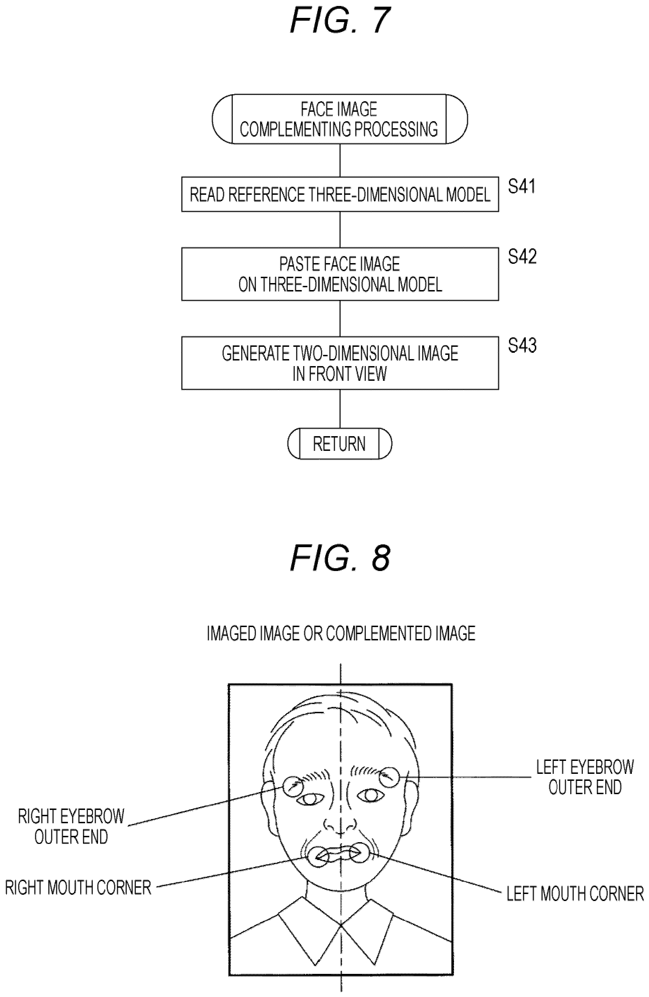

Fig. 7 is a flowchart illustrating a procedure of the face image complementing processing performed by the server device 1 according to the first embodiment. The face image complementing processing illustrated in this flowchart is processing executed at step S23 in a case where it is determined in the flowchart illustrated in Fig. 7 that the face image acquired from the terminal device 3 is not of the front view. The three-dimensional model generation unit 11b of the processing unit 11 of the server device 1 reads the reference three-dimensional model stored in the reference information storage unit 12b (step S41).

The three-dimensional model generation unit 11b pastes the face image of the subject imaged by the camera 36 of the terminal device 3 on the reference three-dimensional model read at step S41, thereby generating the three-dimensional model of a current face of the subject (step S42). At that time, the three-dimensional model generation unit 11b extracts the feature points from the face image, for example, and changes the expression of the reference three-dimensional model or selects the reference three-dimensional model with the most matched feature points from the reference three-dimensional models of a plurality of expressions so that the feature points of the face of the reference three-dimensional model and the feature points of the face image match.

The face image complementing unit 11c of the processing unit 11 generates the two-dimensional image in the front view from the three-dimensional model of the face of the subject generated at step S42 to generate the face image in which the missing portion of the face image imaged by the terminal device 3 is complemented (step S43), and ends the face image complementing processing. For example, the face image complementing unit 11c sets the virtual camera in front of the three-dimensional model of the face of the subject in the three-dimensional virtual space, and acquires the two-dimensional image obtained by imaging the three-dimensional model by the virtual camera, thereby obtaining the complemented face image.

Note that, in this embodiment, the face image of the subject to be subjected to the face image complementing processing is a face image including at least one feature point indicating a feature such as face distortion in a case where the subject has neuropathy. For example, as for a subject in which a feature such as distortion appears in a right half of the face, a face image in which only a left half of the face is imaged is not a target of the face image complementing processing. With the face image in which the left half of the face and at least a part of the right half of the face (a part in which the feature such as the distortion appears) are imaged, it is possible to generate the face image in the front view in which the distortion and the like appears in the right half of the face by the face image complementing processing by selecting the reference three-dimensional model on the basis of the feature points included in the part.

Conversely, for example, as for the subject in which the feature such as the distortion appears in the right half of the face, on the basis of the face image in which the right half of the face with distortion and a part of the left half of the face without distortion are imaged, the face image complementing unit 11c can complement the left half of the face without distortion to generate the face image in the front view with distortion in the right half of the face and no distortion in the left half.

Fig. 8 is a schematic diagram for illustrating a method of estimating the presence or absence of neuropathy performed by the server device 1 according to this embodiment. Fig. 8 illustrates an example of the face image of the subject who can be estimated to have neuropathy. The face image is the image imaged by the camera 36 of the terminal device 3 or the image obtained by complementing the missing portion on the basis of the imaged image. The neuropathy estimation unit 11d of the processing unit 11 of the server device 1 extracts the feature points from the face image of the subject. In the face image in Fig. 8, the feature points of the mouth corner and the eyebrow outer end extracted from the face image of the subject are illustrated.

The neuropathy estimation unit 11d divides the subject into right and left two parts on a median plane (the center line indicated by dashed line in Fig. 8), compares a positional relationship of the feature points between the right half and the left half of the face of the subject, and determines bilateral symmetry of the face of the subject. In Fig. 8, for example, in a case where the positional relationship between a position of the right mouth corner and a position of the left mouth corner of the subject is compared, the position of the right mouth corner is lower than the position of the left mouth corner. Similarly, a position of the right eyebrow outer end of the subject is lower than a position of the left eyebrow outer end. The neuropathy estimation unit 11d calculates a difference in coordinates or a distance from the center line of the feature points present on the right and left sides of the face such as the mouth corner and the eyebrow outer end, and can estimate that there is neuropathy in a case where the difference exceeds a predetermined threshold.

Although not illustrated, the neuropathy estimation unit 11d compares the reference face image of the subject in the normal state imaged in advance with the face image of the subject imaged by the camera 36 of the terminal device 3 or the face image obtained by complementing the same to estimate the presence or absence of neuropathy of the subject. The neuropathy estimation unit 11d extracts the feature points from the reference face image stored in the reference information storage unit 12b, extracts the feature points from the face image acquired from the terminal device 3, and compares the feature points between both images. The neuropathy estimation unit 11d can estimate that there is neuropathy in a case where the difference in position of the corresponding feature points in both images exceeds a predetermined threshold.

### <Display Processing>

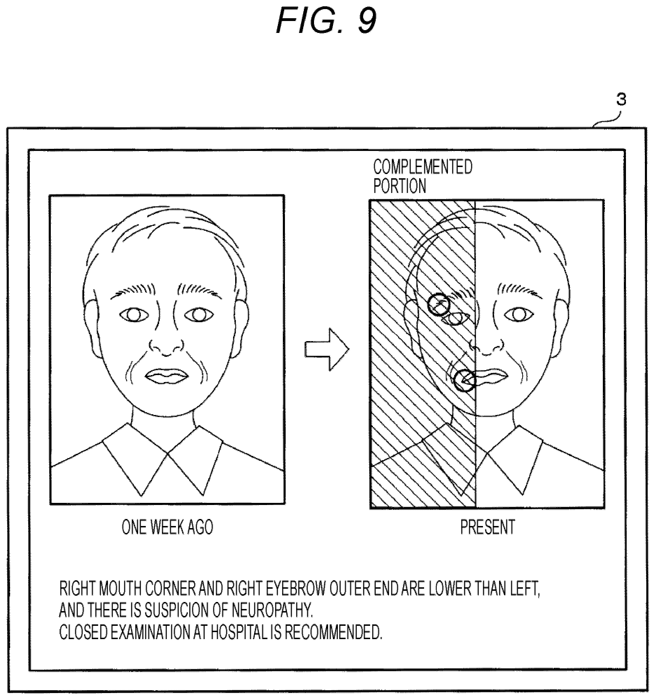

In the information processing system according to this embodiment, in a case where the server device 1 estimates that the subject has neuropathy, this displays information on the terminal device 3 to notify the subject. Fig. 9 is a schematic diagram illustrating an example of a notification screen of the estimation result of neuropathy. Note that, the notification screen illustrated in Fig. 9 may be displayed, for example, after the estimation processing based on the face image is performed and before the diagnostic test is performed, or may be displayed, for example, after the diagnostic test ends. The server device 1 transmits information of an image, a message and the like for displaying the notification screen to the terminal device 3 at an appropriate timing, and the terminal device 3 that receives the same displays the illustrated notification screen on the display unit 34.

On the basis of the information from the server device 1, the terminal device 3 displays, for example, a face image of one week ago of the subject and a current face image used for estimation side by side as illustrated in Fig. 9. At that time, in a case where the missing portion of the face image imaged by the camera 36 is complemented, the terminal device 3 displays the current face image so that the portion actually imaged and the complemented portion of the current face image can be distinguished. As the distinguishable display, for example, a display method such as displaying by changing colors or displaying with shading can be adopted. In the example illustrated in Fig. 9, the current face image is displayed while shading the complemented portion.

As a result of the estimation of neuropathy by the server device 1, the terminal device 3 displays, for example, a message such as "The right mouth corner and right eyebrow outer end are lower than the left, and there is a suspicion of neuropathy. Closed examination at hospital is recommended." below the two face images. On the current face image, a display indicating which site (feature point) of the face is the basis for estimating that there is neuropathy is made. In the example illustrated in Fig. 9, the terminal device 3 displays a figure of a circle surrounding the right mouth corner and the right eyebrow outer end to be superimposed on the face image as the basis of the estimation that there is neuropathy. Note that the method of displaying the basis site is not limited to the superimposition of the circle illustrated in Fig. 9, and may be, for example, display such as coloring or shading, a display method different from that in other sites, or any other display method.

### <Information Collection Processing>

In the information processing system according to this embodiment, the terminal device 3 installed in the home and the like of the subject continuously images the subject. The server device 1 may estimate the presence or absence of neuropathy of the subject on the basis of the face image of the subject imaged by the terminal device 3, and the server device 1 may store the face image used when it is estimated that there is no neuropathy. The server device 1 can store and collect the face image of the subject in the normal state (state without neuropathy), and use the collected face image as the reference face image in subsequent estimation processing.

Moreover, the terminal device 3 may continuously perform measurement by the ranging sensor 37, and the server device 1 may store and collect the measurement result by the ranging sensor 37 together with the face image of the subject in the normal state. The server device 1 can collect the face image by the camera 36 and the measurement result (that is, the surface shape of the face of the subject) of the ranging sensor 37, and generate the three-dimensional model of the face of the subject on the basis of the collected information. The server device 1 continuously collects these pieces of information, periodically generates the three-dimensional model of the face of the subject, and stores the collected face image and the generated three-dimensional model as the reference face image and the reference three-dimensional model of the subject in the reference information storage unit 12b.

The collection of the information of the face image and the surface shape by the server device 1 does not target all the pieces of information acquired from the terminal device 3, and may be, for example, about several pieces per day. For example, the server device 1 stores about several pieces of information selected under various conditions from information of a plurality of face images imaged by the terminal device 3 in one day and the surface shapes. As the selection condition, various conditions such as the front view of the subject, for example, brightness of the image exceeding a predetermined threshold, for example, a ratio of the face with respect to an entire imaged image exceeding a predetermined threshold, for example, a predetermined expression such as a smile, for example, and the like can be adopted. Note that the selection condition may be any condition.

The generation of the reference three-dimensional model by the server device 1 may be performed at a predetermined cycle such as once every several months. At that time, the server device 1 may overwrite the already stored old reference face image and reference three-dimensional model with new reference face image and reference three-dimensional model to update, or may add and store the new reference face image and reference three-dimensional model while leaving the old reference face image and reference three-dimensional model.

### <Summary>

In the information processing system according to this embodiment having the above-described configuration, the server device 1 acquires the face image (face information) of the subject imaged (detected) by the camera 36 (sensor) of the terminal device 3, generates the three-dimensional model (face structure information) of the face of the subject on the basis of the acquired face image, complements the missing portion of the face image on the basis of the generated three-dimensional model, and estimates the presence or absence of neuropathy of the subject on the basis of the complemented face image. As a result, the information processing system can be expected to accurately estimate the presence or absence of neuropathy of the subject on the basis of the face image imaged in a direction other than the front view. Since the positional relationship between the subject and the device such as the camera that images the face of the subject is not limited, it can be expected that the restriction of the place where the device is installed in the house or the like is alleviated, and it can be expected that watching, abnormality detection and the like of the subject in real time are performed.

In the information processing system according to this embodiment, the server device 1 stores the reference three-dimensional model of the subject generated in advance in the reference information storage unit 12b, and generates the three-dimensional model of the subject on the basis of the face image imaged by the camera 36 of the terminal device 3 and the reference three-dimensional model stored in the reference information storage unit 12b. The reference three-dimensional model is generated on the basis of a multi-view face image (multi-view face information) obtained by imaging the face of the subject in two or more directions by a camera and a shape model (three-dimensional face information) reproduced by measuring the surface shape of the face of the subject with a ranging sensor. Note that the server device 1 may store the multi-view face image and the shape model for generating the reference three-dimensional model and generate the reference three-dimensional model as necessary, instead of generating and storing the reference three-dimensional model in advance. As a result, the information processing system can be expected to accurately generate the reference three-dimensional model and accurately complement the missing portion of the face image.

In the information processing system according to this embodiment, the server device 1 stores and accumulates the face image of the subject imaged by the camera 36 by the terminal device 3 and the shape information of the face of the subject measured by the ranging sensor 37 in the storage unit 12. On the basis of the accumulated information, the server device 1 can update the reference three-dimensional model and the like stored in the reference information storage unit 12b, and can be expected to accurately complement the missing portion of the face image by using the updated reference three-dimensional model.

In the information processing system according to this embodiment, the server device 1 estimates the presence or absence of neuropathy of the subject on the basis of a comparison result between the right half of the face image of the subject imaged by the terminal device 3 or the face image obtained by complementing the same and the right half of the face image stored in the reference information storage unit 12b, and a comparison result between the left half of the face image of the subject imaged by the terminal device 3 or the face image obtained by complementing the same and the left half of the face image stored in the reference information storage unit 12b. For example, in a case where the right half of the complemented face image is lower than the right half of the face image stored in the reference information storage unit 12b, or in a case where the left half of the complemented face image is lower than the left half of the face image stored in the reference information storage unit 12b, the server device 1 can estimate that the subject has neuropathy. As a result, the information processing system according to this embodiment can be expected to accurately estimate the presence or absence of neuropathy of the subject.

In the information processing system according to this embodiment, the presence or absence of neuropathy of the subject is estimated on the basis of symmetry between the right half and the left half of the face of the subject on the basis of the face image of the subject imaged by the terminal device 3 or the face image obtained by complementing the same. For example, in a case where the right half of the face is lower than the left half, or in a case where the left half of the face is lower than the right half, the server device 1 can estimate that the subject has neuropathy. As a result, the information processing system according to this embodiment can be expected to accurately estimate the presence or absence of neuropathy of the subject.

In the information processing system according to this embodiment, in a case where the face image imaged by the terminal device 3 is a face image other than the face image in the front view, the complemented face image in the front view is displayed on the display unit of the terminal device 3. The terminal device 3 displays the complemented face image and the reference face image stored in the reference information storage unit 12b side by side. Note that, the terminal device 3 may superimpose and display the complemented face image and the reference face image instead of displaying them side by side. The terminal device 3 displays which part is the basis of the estimation result of the presence or absence of the neuropathy together with the face image of the subject. As a result, the information processing system according to this embodiment can be expected to present the estimation result of the presence or absence of neuropathy and the like to the subject and the like.

In the information processing system according to this embodiment, in a case where the server device 1 estimates that the subject has neuropathy on the basis of the face image of the subject imaged by the camera 36 of the terminal device 3, the terminal device 3 measures the surface shape of the face of the subject by the ranging sensor 37, and the server device 1 further estimates the presence or absence of the neuropathy of the subject on the basis of the measured surface shape. Note that, the information processing system may perform estimation based on the measurement result of the ranging sensor 37 first and perform estimation based on the imaged image of the camera 36 later. In a case where it is estimated that the subject has neuropathy by the estimation processing, the information processing system according to this embodiment performs the diagnostic test on the subject. As a result, the information processing system according to this embodiment can be expected to accurately determine the presence or absence, the degree and the like of neuropathy of the subject.

Note that, in this embodiment, the camera 36 is used as the sensor for acquiring the face information of the subject in the first estimation processing of the presence or absence of neuropathy, but this is merely an example, and there is no limitation; for example, the sensor may be the ranging sensor 37, or may be a sensor other than them. In this embodiment, the ranging sensor 37 is used as a second sensor in second estimation processing of the presence or absence of neuropathy, but this is an example and there is no limitation. The second sensor may be, for example, a microphone, an event-driven imaging device that extracts a change in luminance, a millimeter wave sensor, an ultrasonic sensor, a thermographic camera or the like, or may be a sensor other than them. The second sensor may include a plurality of types of sensors. The information acquired by the second sensor is not limited to the face information, and may be information indicating abnormality of the subject such as a change in arm fluctuation (body shaking/shivering), weakness of the arm, and speech abnormality (aphasia/dysarthria).

In the information processing system according to this embodiment, the server device 1 performs the processing of estimating the presence or absence of neuropathy of the subject on the basis of the face image imaged by the terminal device 3 by the camera; however, there is no limitation, and the terminal device 3 can perform the processing of estimating the presence or absence of neuropathy, and in this case, the information processing system is not required to include the server device 1.

### <Second Embodiment>

In the information processing system according to the first embodiment, the server device 1 complements the missing portion of the face image of the subject imaged by the terminal device 3 using the reference three-dimensional model and the reference face image stored in advance. In contrast, in an information processing system according to a second embodiment, a server device 1 complements a missing portion of a face image of a subject imaged by a terminal device 3 by using a learning model subjected to machine learning in advance (so-called artificial intelligence (AI)) without storing reference information such as a reference three-dimensional model and a reference face image in advance.

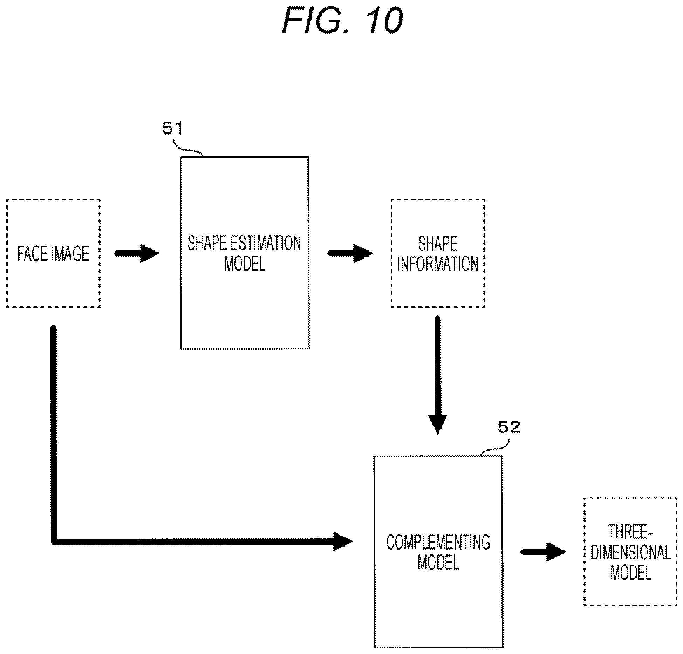

Fig. 10 is a schematic diagram for illustrating a configuration of the learning model included in the server device 1 according to the second embodiment. In the server device 1 according to the second embodiment, a reference information storage unit 12b is not provided in a storage unit 12, and in place of this, information regarding a learned learning model is stored in the storage unit 12. The information regarding the learning model can include, for example, information defining a structure of the learning model and information such as a value of an internal parameter determined by the machine learning. The server device 1 according to the second embodiment performs processing of complementing the missing portion of the face image of the subject using two learning models of a shape estimation model 51 and a complementing model 52.

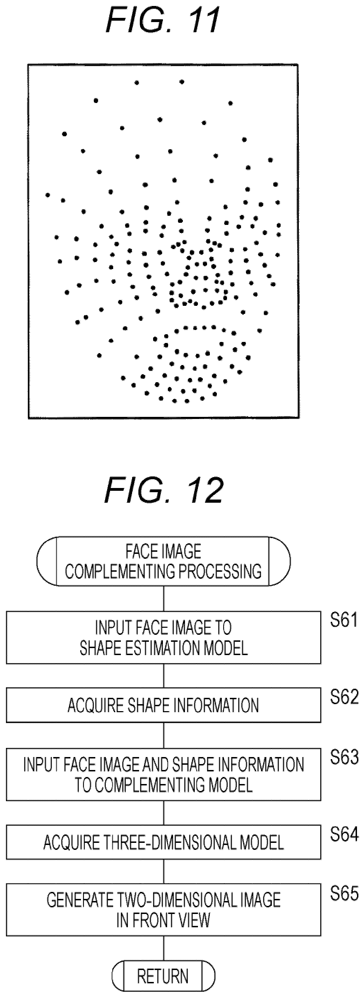

The shape estimation model 51 is the learning model subjected to the machine learning in advance so as to receive a two-dimensional face image as an input, estimate a three-dimensional surface shape of a face imaged in the face image, and output an estimation result as shape information. For example, a learned learning model of a face mesh might be used as the shape estimation model 51. The learning model of the face mesh is a machine learning model that detects key points (feature points) of a face from an image, and can output hundreds of feature points from a human face in three-dimensional coordinates. Fig. 11 is a schematic diagram illustrating an example of shape information output by the shape estimation model 51. This diagram is obtained by plotting a plurality of feature points output from the learning model of the face mesh in a three-dimensional virtual space in which the shape of the human face is reproduced, and a face direction and the like can be changed in the three-dimensional virtual space. Note that, since the learning model of the face mesh is an existing technology, detailed description of a machine learning method and the like is omitted. A learning model other than the learning model of the face mesh may be adopted as the shape estimation model 51.

Note that, in this embodiment, in a case where the subject has neuropathy, the shape estimation model 51 can output shape information that reproduces face distortion or the like of the subject by inputting the face image including at least one feature point indicating the feature such as the face distortion. Even in a case where the subject has neuropathy, in a case where a face image including no feature point indicating a feature such as face distortion at all is input, the shape estimation model 51 does not reproduce the face distortion and the like of the subject and can output shape information reproducing the face in a normal state.

The complementing model 52 is a learning model subjected to machine learning in advance so as to receive the two-dimensional face image and the shape information estimated by the shape estimation model 51 from the face image as an input, and generate the three-dimensional model of the face imaged in the face image. The three-dimensional model output from the complementing model 52 is the three-dimensional model obtained by pasting the face image on the input shape information and complementing the missing portion of the face image. As the complementing model 52, for example, a learning model such as a deep neural network (DNN) or a convolutional neural network (CNN) can be adopted. The complementing model 52 can be generated, for example, by acquiring the face image, shape information, and three-dimensional model by a procedure (refer to Fig. 5) similar to that in generation of the three-dimensional model performed as the pre-processing in the information processing system according to the first embodiment and performing the machine learning using them as teacher data. Note that, the machine learning processing of these learning models is an existing technology, so that detailed description thereof will be omitted.

Fig. 12 is a flowchart illustrating a procedure of face image complementing processing performed by the server device 1 according to the second embodiment. A three-dimensional model generation unit 11b of a processing unit 11 of the server device 1 according to the second embodiment inputs the face image of the subject imaged by a camera 36 of the terminal device 3 to the shape estimation model 51 subjected to machine learning in advance (step S61). The three-dimensional model generation unit 11b acquires the shape information output from the shape estimation model 51 (step S62).

Next, the three-dimensional model generation unit 11b inputs the face image of the subject and the shape information acquired at step S62 to the complementing model 52 (step S63). The three-dimensional model generation unit 11b generates a three-dimensional model of the face of the subject by acquiring the three-dimensional model output from the complementing model 52 (step S64). The face image complementing unit 11c of the processing unit 11 generates the two-dimensional image in the front view from the three-dimensional model of the face of the subject generated at step S63 to generate the face image in which the missing portion of the face image imaged by the terminal device 3 is complemented (step S65), and ends the face image complementing processing.

In the information processing system according to the second embodiment, similarly to the information processing system according to the first embodiment, the face image imaged by the terminal device 3 by the camera 36 and/or shape information measured by the ranging sensor 37 are stored to be accumulated. These pieces of accumulated information can be used for relearning of the complementing model 52. The server device 1 can store information at a frequency of several times a day, for example, and can perform relearning of the complementing model 52 at a frequency of once a week, for example.

In a case where the server device 1 according to the second embodiment complements the face image of the subject imaged by the camera 36 of the terminal device 3, estimates that there is neuropathy on the basis of the complemented face image, and then, for example, estimates that there is no neuropathy without performing complementing processing on the basis of the face image in the front view imaged by the camera 36 of the terminal device 3 within a predetermined time, this determines that the first estimation result is erroneous. The server device 1 stores, as information for relearning, information such as a face image used for erroneous first estimation processing, a face image obtained by complementing the same, a result of measurement performed by the ranging sensor 37 subsequent to the first estimation processing, and a face image used for later estimation processing. In this manner, by accumulating information in a case where the server device 1 determines that there is an error in the estimation result and using the information for relearning of the complementing model 52, it can be expected to improve the accuracy of complementation of the face image by the complementing model 52.

In the information processing system according to the second embodiment having the above-described configuration, the server device 1 includes the learning model (learning model obtained by combining the shape estimation model 51 and the complementing model 52) subjected to machine learning so as to receive, as the input, the face image obtained by imaging the face of the subject and output the three-dimensional model of the subject. The server device 1 inputs the face image imaged by the camera 36 of the terminal device 3 to the learning model and acquires the three-dimensional model output by the learning model, thereby generating the three-dimensional model from the face image of the subject. As a result, the information processing system according to the second embodiment can be expected to accurately generate the three-dimensional model of the subject and accurately complement the missing portion of the face image.

It is supposed that the embodiments disclosed herein are considered to be an example in all respects and not to be restrictive. The scope of the present disclosure is indicated not by the above meaning but by claims and is intended to include all changes within the meaning and scope equivalent to claims.

### Reference Signs List

- 1: Server device
- 3: Terminal device
- 11: Processing unit
- 11a: Face image acquisition unit
- 11b: Three-dimensional model generation unit
- 11c: Face image complementing unit
- 11d: Neuropathy estimation unit
- 11e: Second estimation unit
- 11f: Diagnostic test processing unit
- 11g: Notification processing unit
- 12: Storage unit
- 12a: Server program
- 12b: Reference information storage unit
- 13: Communication unit
- 31: Processing unit
- 31a: Imaging processing unit
- 31b: Ranging processing unit
- 31c: Diagnostic test processing unit
- 32: Storage unit
- 32a: Program
- 33: Communication unit
- 34: Display unit
- 35: Operation unit
- 36: Camera
- 37: Ranging sensor
- 51: Shape estimation model
- 52: Complementing model
- 98, 99: Recording medium
- N: Network

## Claims

1. A computer program that allows a computer to:
acquire face information of a subject detected by a sensor;
generate face structure information of the subject on a basis of the acquired face information;
complement missing face information of the subject with respect to the acquired face information on a basis of the generated face structure information; and
estimate presence or absence of neuropathy of the subject on a basis of the complemented face information.

2. The computer program according to claim 1, that
acquires reference face structure information of the subject generated in advance from a storage unit; and
generates face structure information of the subject on a basis of the face information detected by the sensor and the reference face structure information stored in the storage unit,
wherein preferably the reference face structure information includes multi-view face information acquired by a sensor in two or more directions with respect to a front face of the face of the subject.

3. The computer program according to claim 2, wherein
the reference face structure information includes three-dimensional face information obtained by acquiring a three-dimensional shape of the face of the subject by a sensor.

4. The computer program according to any one of claims 2 to 3, wherein
the reference face structure information includes face structure information generated on a basis of the multi-view face information acquired by a sensor in two or more directions with respect to a front face of the face of the subject and/or face structure information generated on a basis of the three-dimensional face information obtained by acquiring a three-dimensional shape of the face of the subject by a sensor.

5. The computer program according to any one of claims 2 to 4, that
stores the acquired face information or the face structure information generated on a basis of the face information in the storage unit as the reference face structure information.

6. The computer program according to any one of claims 2 to 5, that
estimates the presence or absence of neuropathy of the subject on a basis of a comparison result between a right half of the face of the subject based on the complemented face information and a right half of the face based on the reference face structure information and a comparison result between a left half of the face of the subject based on the complemented face information and a left half of the face based on the reference face structure information,
wherein preferably the computer program estimates that the subject has neuropathy in a case where the right half of the face based on the complemented face information is lower than the right half of the face based on the reference face structure information, or in a case where the left half of the face based on the complemented face information is lower than the left half of the face based on the reference face structure information.

7. The computer program according to any one of claims 1 to 6, that
generates face structure information of the subject by inputting the face information detected by the sensor to a learning model subjected to machine learning so as to receive the face information of the subject as an input and output the face structure information of the subject and acquiring the face structure information output by the learning model.

8. The computer program according to any one of claims 1 to 7, that
estimates the presence or absence of neuropathy of the subject on a basis of symmetry of a right half and a left half of the face of the subject based on the complemented face information,
wherein preferably the computer program estimates that the subject has neuropathy in a case where the right half of the face is lower than the left half, or in a case where the left half of the face is lower than the right half.

9. The computer program according to any one of claims 1 to 8, that
stores, in a case where it is estimated that the subject has neuropathy on a basis of face information obtained by complementing the face information detected by the sensor, and then it is estimated that the subject does not have neuropathy without complementing on a basis of the face information detected by the sensor, the face information when estimating that there is neuropathy in the storage unit.

10. The computer program according to any one of claims 1 to 9, that
in a case where the face information detected by the sensor is face information other than a front view of the subject, displays complemented face information in a front view on a display unit, wherein preferably
the computer program displays the complemented face information and face information of the subject stored in advance in the storage unit on the display unit side by side or in a superimposed manner and/or
the computer program displays a portion involved in an estimation result of the presence or absence of neuropathy on the display unit together with the face information.

11. The computer program according to any one of claims 1 to 10, that either
acquires information of the subject by a second sensor different from the sensor in a case where it is estimated that the subject has neuropathy; and
further estimates the presence or absence of neuropathy of the subject on a basis of the information acquired by the second sensor or else
acquires information of the subject by a second sensor different from the sensor;
estimates the presence or absence of neuropathy of the subject on a basis of the information acquired by the second sensor; and
in a case where it is estimated that the subject has neuropathy, further estimates the presence or absence of neuropathy of the subject on a basis of the face information acquired by the sensor.

12. The computer program according to any one of claims 1 to 11, that
performs a diagnostic test on the subject in a case where it is estimated that the subject has neuropathy.

13. The computer program according to any one of claims 1 to 12,
wherein the sensor is a camera that images a two-dimensional image
or wherein the sensor is a ranging sensor.

14. An information processing device comprising:
an acquisition unit that acquires face information of a subject detected by a sensor;
a generation unit that generates face structure information of the subject on a basis of the acquired face information;
a complementing unit that complements missing face information of the subject with respect to the acquired face information on a basis of the generated face structure information; and
an estimation unit that estimates presence or absence of neuropathy of the subject on a basis of the complemented face information.

## Patentansprüche

1. Computerprogramm, das es einem Computer ermöglicht:
eine Gesichtsinformation eines Subjekts entgegenzunehmen, die von einem Sensor erfasst wird;
eine Gesichtsstrukturinformation des Subjekts basierend auf der erfassten Gesichtsinformation zu erzeugen;
eine fehlende Gesichtsinformation des Subjekts in Bezug auf die entgegen genommene Gesichtsinformation basierend auf der erzeugten Gesichtsstrukturinformation zu ergänzen; und
das Vorhandensein oder Fehlen einer Neuropathie des Subjekts basierend auf der ergänzten Gesichtsinformation abzuschätzen.

2. Computerprogramm nach Anspruch 1, das
eine im Vorhinein erzeugte Referenz-
Gesichtsstrukturinformation des Subjekts von einer Speichereinheit entgegennimmt; und
eine Gesichtsstrukturinformation des Subjekts basierend auf der von dem Sensor erfassten Gesichtsinformation und der in der Speichereinheit gespeicherten Referenz-Gesichtsstrukturinformation erzeugt,
wobei vorzugsweise die Referenz-Gesichtsstrukturinformation eine Mehrfachansicht-Gesichtsinformation beinhaltet, die von einem Sensor in zwei oder mehr Richtungen in Bezug auf eine Vorderseite des Gesichts des Subjekts erfasst wird.

3. Computerprogramm nach Anspruch 2, wobei
die Referenz-Gesichtsstrukturinformation eine dreidimensionale Gesichtsinformation beinhaltet, die durch Erfassen einer dreidimensionalen Form des Gesichts des Subjekts durch einen Sensor erhalten wird.

4. Computerprogramm nach einem der Ansprüche 2 bis 3, wobei
die Referenz-Gesichtsstrukturinformation eine Gesichtsstrukturinformation beinhaltet, die basierend auf der Mehrfachansicht-Gesichtsinformation erzeugt wird, die von einem Sensor in zwei oder mehr Richtungen in Bezug auf eine Vorderseite des Gesichts des Subjekts erfasst wird, und/oder eine Gesichtsstrukturinformation beinhaltet, die basierend auf der dreidimensionalen Gesichtsinformation erzeugt wird, die durch Erfassen einer dreidimensionalen Form des Gesichts des Subjekts durch einen Sensor erhalten wird.

5. Computerprogramm nach einem der Ansprüche 2 bis 4, das
die entgegen genommene Gesichtsinformation oder die Gesichtsstrukturinformation, die basierend auf der Gesichtsinformation erzeugt wird, in der Speichereinheit als die Referenz-Gesichtsstrukturinformation speichert.

6. Computerprogramm nach einem der Ansprüche 2 bis 5, das
das Vorhandensein oder Fehlen einer Neuropathie des Subjekts basierend auf einem Vergleichsergebnis zwischen einer rechten Gesichtshälfte des Subjekts basierend auf der ergänzten Gesichtsinformation und einer rechten Gesichtshälfte basierend auf der Referenz-Gesichtsstrukturinformation und einem Vergleichsergebnis zwischen einer linken Gesichtshälfte des Subjekts basierend auf der ergänzten Gesichtsinformation und einer linken Gesichtshälfte basierend auf der Referenz-Gesichtsstrukturinformation abschätzt,
wobei vorzugsweise das Computerprogramm in einem Fall, in dem die rechte Gesichtshälfte basierend auf der ergänzten Gesichtsinformation niedriger ist als die rechte Gesichtshälfte basierend auf der Referenz-Gesichtsstrukturinformation, oder in einem Fall, in dem die linke Gesichtshälfte basierend auf der ergänzten Gesichtsinformation niedriger ist als die linke Gesichtshälfte basierend auf der Referenz-Gesichtsstrukturinformation, abschätzt, dass das Subjekt eine Neuropathie hat.

7. Computerprogramm nach einem der Ansprüche 1 bis 6, das
eine Gesichtsstrukturinformation des Subjekts erzeugt, indem die von dem Sensor erfasste Gesichtsinformation in ein Lernmodell eingegeben wird, das maschinellem Lernen unterzogen wird, um die Gesichtsinformation des Subjekts als Eingabe zu erhalten und die Gesichtsstrukturinformation des Subjekts auszugeben, und die von dem Lernmodell ausgegebene Gesichtsstrukturinformation entgegennimmt.

8. Computerprogramm nach einem der Ansprüche 1 bis 7, das
das Vorhandensein oder Fehlen einer Neuropathie des Subjekts basierend auf einer Symmetrie einer rechten Gesichtshälfte und einer linken Gesichtshälfte des Subjekts auf der Grundlage der ergänzten Gesichtsinformation abschätzt,
wobei vorzugsweise das Computerprogramm in einem Fall, in dem die rechte Gesichtshälfte niedriger ist als die linke Gesichtshälfte, oder in einem Fall, in dem die linke Gesichtshälfte niedriger ist als die rechte Gesichtshälfte, abschätzt, dass das Subjekt eine Neuropathie hat.

9. Computerprogramm nach einem der Ansprüche 1 bis 8, das
in einem Fall, in dem basierend auf einer Gesichtsinformation, die durch Ergänzen der von dem Sensor erfassten Gesichtsinformation erhalten wird, abgeschätzt wird, dass das Subjekt eine Neuropathie hat, und dann ohne Ergänzung basierend auf der von dem Sensor erfassten Gesichtsinformation abgeschätzt wird, dass das Subjekt keine Neuropathie hat, die Gesichtsinformation in der Speichereinheit speichert, aufgrund derer abgeschätzt wird, dass eine Neuropathie vorliegt.

10. Computerprogramm nach einem der Ansprüche 1 bis 9, das
in einem Fall, in dem die von dem Sensor erfasste Gesichtsinformation eine andere Gesichtsinformation als eine Vorderansicht des Subjekts ist, eine ergänzte Gesichtsinformation in einer Vorderansicht auf einer Anzeigeeinheit anzeigt, wobei vorzugsweise
das Computerprogramm die ergänzte Gesichtsinformation und die im Vorhinein in der Speichereinheit gespeicherte Gesichtsinformation des Subjekts auf der Anzeigeeinheit nebeneinander oder in einer überlagerten Weise anzeigt und/oder
das Computerprogramm einen Abschnitt, der an einem Abschätzungsergebnis des Vorhandenseins oder Fehlens einer Neuropathie beteiligt ist, zusammen mit der Gesichtsinformation auf der Anzeigeeinheit anzeigt.

11. Computerprogramm nach einem der Ansprüche 1 bis 10, das entweder
in einem Fall, in dem abgeschätzt wird, dass das Subjekt eine Neuropathie aufweist, eine Information über das Subjekt entgegennimmt, die durch einen zweiten Sensor erfasst wird, der sich von dem Sensor unterscheidet; und
weiterhin das Vorhandensein oder Fehlen einer Neuropathie des Subjekts basierend auf der Information abschätzt, die durch den zweiten Sensor erfasst wurde, oder
eine Information über das Subjekt durch einen zweiten Sensor erfasst, der sich von dem Sensor unterscheidet;
das Vorhandensein oder Fehlen einer Neuropathie des Subjekts basierend auf der Information abschätzt, die durch den zweiten Sensor erfasst wurde; und
in einem Fall, in dem abgeschätzt wird, dass das Subjekt eine Neuropathie aufweist, weiterhin das Vorhandensein oder Fehlen einer Neuropathie des Subjekts basierend auf der Gesichtsinformation abschätzt, die durch den Sensor erfasst wurde.

12. Computerprogramm nach einem der Ansprüche 1 bis 11, das
in einem Fall, in dem abgeschätzt wird, dass das Subjekt eine Neuropathie aufweist, einen diagnostischen Test an dem Subjekt durchführt.

13. Computerprogramm nach einem der Ansprüche 1 bis 12,
wobei der Sensor eine Kamera ist, die ein zweidimensionales Bild erzeugt,
oder wobei der Sensor ein Entfernungsmesssensor ist.

14. Informationsverarbeitungsvorrichtung, die aufweist:
eine Erfassungseinheit, die eine Gesichtsinformation eines Subjekts entgegennimmt, die von einem Sensor erfasst wurde;
eine Erzeugungseinheit, die eine Gesichtsstrukturinformation des Subjekts basierend auf der entgegen genommenen Gesichtsinformation erzeugt;
eine Ergänzungseinheit, die eine fehlende Gesichtsinformation des Subjekts in Bezug auf die entgegen genommene Gesichtsinformation basierend auf der erzeugten Gesichtsstrukturinformation ergänzt; und
eine Abschätzungseinheit, die das Vorhandensein oder Fehlen einer Neuropathie des Subjekts basierend auf der ergänzten Gesichtsinformation abschätzt.

## Revendications

1. Programme informatique qui permet à un ordinateur de :
acquérir des informations de visage d'un sujet détectées par un capteur ;
générer des informations de structure de visage du sujet sur la base des informations de visage acquises ;
compléter des informations de visage manquantes du sujet par rapport aux informations de visage acquises sur la base des informations de structure de visage générées ; et
estimer la présence ou l'absence d'une neuropathie du sujet sur la base des informations de visage complétées.

2. Programme informatique selon la revendication 1, qui
acquiert des informations de structure de visage de référence du sujet générées à l'avance à partir d'une unité de stockage ; et
génère des informations de structure de visage du sujet sur la base des informations de visage détectées par le capteur et des informations de structure de visage de référence stockées dans l'unité de stockage,
où, de préférence, les informations de structure de visage de référence comprennent des informations de visage multi-vues acquises par un capteur dans deux directions ou plus par rapport à une face avant du visage du sujet.

3. Programme informatique selon la revendication 2, où
les informations de structure de visage de référence comprennent des informations de visage tridimensionnel obtenues en acquérant une forme tridimensionnelle du visage du sujet par un capteur.

4. Programme informatique selon l'une quelconque des revendications 2 à 3, où
les informations de structure de visage de référence comprennent des informations de structure de visage générées sur la base des informations de visage multi-vues acquises par un capteur dans deux directions ou plus par rapport à une face avant du visage du sujet et/ou des informations de structure de visage générées sur la base des informations de visage tridimensionnel obtenues en acquérant une forme tridimensionnelle du visage du sujet par un capteur.

5. Programme informatique selon l'une quelconque des revendications 2 à 4, qui
stocke les informations de visage acquises ou les informations de structure de visage générées sur la base des informations de visage dans l'unité de stockage en tant qu'informations de structure de visage de référence.

6. Programme informatique selon l'une quelconque des revendications 2 à 5, qui
estime la présence ou l'absence d'une neuropathie du sujet sur la base d'un résultat de comparaison entre une moitié droite du visage du sujet sur la base des informations de visage complétées et une moitié droite du visage sur la base des informations de structure de visage de référence et d'un résultat de comparaison entre une moitié gauche du visage du sujet sur la base des informations de visage complétées et une moitié gauche du visage sur la base des informations de structure de visage de référence,
où, de préférence, le programme informatique estime que le sujet présente une neuropathie dans un cas où la moitié droite du visage sur la base des informations de visage complétées est inférieure à la moitié droite du visage sur la base des informations de structure de visage de référence, ou dans un cas où la moitié gauche du visage sur la base des informations de visage complétées est inférieure à la moitié gauche du visage sur la base des informations de structure de visage de référence.

7. Programme informatique selon l'une quelconque des revendications 1 à 6, qui
génère des informations de structure de visage du sujet en entrant les informations de visage détectées par le capteur dans un modèle d'apprentissage soumis à un apprentissage machine de manière à recevoir les informations de visage du sujet en tant qu'entrée et à délivrer en sortie les informations de structure de visage du sujet et en acquérant les informations de structure de visage délivrées en sortie par le modèle d'apprentissage.

8. Programme informatique selon l'une quelconque des revendications 1 à 7, qui
estime la présence ou l'absence d'une neuropathie du sujet sur la base d'une symétrie d'une moitié droite et d'une moitié gauche du visage du sujet sur la base des informations de visage complétées,
où, de préférence, le programme informatique estime que le sujet présente une neuropathie dans un cas où la moitié droite du visage est inférieure à la moitié gauche, ou dans un cas où la moitié gauche du visage est inférieure à la moitié droite.

9. Programme informatique selon l'une quelconque des revendications 1 à 8, qui
stocke, dans un cas où il est estimé que le sujet présente une neuropathie sur la base d'informations de visage obtenues en complétant les informations de visage détectées par le capteur, et ensuite il est estimé que le sujet ne présente pas de neuropathie sans compléter sur la base des informations de visage détectées par le capteur, les informations de visage lors de l'estimation qu'il y a une neuropathie dans l'unité de stockage.

10. Programme informatique selon l'une quelconque des revendications 1 à 9, qui
dans un cas où les informations de visage détectées par le capteur sont des informations de visage autres qu'une vue de face du sujet, affiche des informations de visage complétées dans une vue de face sur une unité d'affichage, où, de préférence,
le programme informatique affiche les informations de visage complétées et les informations de visage du sujet stockées à l'avance dans l'unité de stockage sur l'unité d'affichage côte à côte ou de manière superposée et/ou
le programme informatique affiche une portion impliquée dans un résultat d'estimation de la présence ou de l'absence d'une neuropathie sur l'unité d'affichage conjointement avec les informations de visage.

11. Programme informatique selon l'une quelconque des revendications 1 à 10, qui soit
acquiert des informations du sujet par un second capteur différent du capteur dans un cas où il est estimé que le sujet présente une neuropathie ; et
estime en outre la présence ou l'absence d'une neuropathie du sujet sur la base des informations acquises par le second capteur, ou bien
acquiert des informations du sujet par un second capteur différent du capteur ; estime la présence ou l'absence d'une neuropathie du sujet sur la base des informations acquises par le second capteur ; et
dans un cas où il est estimé que le sujet présente une neuropathie, estime en outre la présence ou l'absence d'une neuropathie du sujet sur la base des informations de visage acquises par le capteur.

12. Programme informatique selon l'une quelconque des revendications 1 à 11, qui
effectue un test de diagnostic sur le sujet dans un cas où il est estimé que le sujet présente une neuropathie.

13. Programme informatique selon l'une quelconque des revendications 1 à 12, dans lequel le capteur est une caméra qui image une image bidimensionnelle ou dans lequel le capteur est un capteur de télémétrie.

14. Dispositif de traitement d'informations comprenant :
une unité d'acquisition qui acquiert des informations de visage d'un sujet détectées par un capteur ;
une unité de génération qui génère des informations de structure de visage du sujet sur la base des informations de visage acquises ;
une unité de complémentation qui complète des informations de visage manquantes du sujet par rapport aux informations de visage acquises sur la base des informations de structure de visage générées ; et
une unité d'estimation qui estime la présence ou l'absence d'une neuropathie du sujet sur la base des informations de visage complétées.
